# EUROPEAN PATENT SPECIFICATION

(11) **EP 2 235 177 B1**
(45) Date of publication and mention of the grant of the patent: **18.07.2012**
(21) Application number: 09761719.5
(22) Date of filing: 09.06.2009
(51) Int. Cl.: C12N 15/10, C12P 19/34, C12N 9/12

(54) **METHOD FOR ENZYMATIC SYNTHESIS OF CHEMICALLY MODIFIED RNA**
VERFAHREN ZUR ENZYMATISCHEN SYNTHESE CHEMISCH MODIFIZIERTER RNA
PROCÉDÉ PERMETTANT LA SYNTHÈSE ENZYMATIQUE DE L'ARN MODIFIÉ CHIMIQUEMENT

(30) Priority: 13.06.2008 EP 08010829
(43) Date of publication of application: 06.10.2010
(73) Proprietor: RiboxX GmbH, 01445 Radebeul (DE)
(72) Inventor: ROHAYEM, Jacques, 01277 Dresden (DE)
(74) Representative: Habermann, Hruschka & Schnabel
(86) International application number: PCT/EP2009/057119
(87) International publication number: WO 2009/150156

(56) References cited:
- WO-A-00/66605
- WO-A-01/46396
- WO-A-02/092774
- WO-A-03/038042
- WO-A-2007/012329
- DUTARTRE HÉLÈNE ET AL: "General catalytic deficiency of hepatitis C virus RNA polymerase with an S282T mutation and mutually exclusive resistance towards 2'-modified nucleotide analogues." ANTIMICROBIAL AGENTS AND CHEMOTHERAPY DEC 2006, vol. 50, no. 12, December 2006 (2006-12), pages 4161-4169, XP002544502 ISSN: 0066-4804
- DUTARTRE HÉLÈNE ET AL: "A relaxed discrimination of 2'-O-methyl-GTP relative to GTP between de novo and Elongative RNA synthesis by the hepatitis C RNA-dependent RNA polymerase NS5B." THE JOURNAL OF BIOLOGICAL CHEMISTRY 25 FEB 2005, vol. 280, no. 8, 25 February 2005 (2005-02-25), pages 6359-6368, XP002544503 ISSN: 0021-9258
- ZAMYATKIN DMITRY F ET AL: "Structural insights into mechanisms of catalysis and inhibition in Norwalk virus polymerase." THE JOURNAL OF BIOLOGICAL CHEMISTRY 21 MAR 2008, vol. 283, no. 12, 21 March 2008 (2008-03-21), pages 7705-7712, XP002544504 ISSN: 0021-9258 cited in the application
- FULLERTON STEPHEN W B ET AL: "Structural and functional characterization of sapovirus RNA-dependent RNA polymerase." JOURNAL OF VIROLOGY FEB 2007, vol. 81, no. 4, February 2007 (2007-02), pages 1858-1871, XP002544505 ISSN: 0022-538X
- ROHAYEM JACQUES ET AL: "Characterization of norovirus 3Dpol RNA-dependent RNA polymerase activity and initiation of RNA synthesis." THE JOURNAL OF GENERAL VIROLOGY SEP 2006, vol. 87, no. Pt 9, September 2006 (2006-09), pages 2621-2630, XP002544506 ISSN: 0022-1317
- ROHAYEM JACQUES ET AL: "Protein-primed and de novo initiation of RNA synthesis by norovirus 3Dpol." JOURNAL OF VIROLOGY JUL 2006, vol. 80, no. 14, July 2006 (2006-07), pages 7060-7069, XP002544507 ISSN: 0022-538X
- BELLIOT GAEL ET AL: "Norovirus proteinase-polymerase and polymerase are both active forms of RNA-dependent RNA polymerase", JOURNAL OF VIROLOGY, THE AMERICAN SOCIETY FOR MICROBIOLOGY, US, vol. 79, no. 4, 1 February 2005 (2005-02-01), pages 2393-2403, XP002581907, ISSN: 0022-538X, DOI: DOI:10.1128/JVI.79.4.2393-2403.2005

### SEQUENCE LISTING

<110> Technische Universität Dresden
<120> Method for enzymatic synthesis of chemically modified RNA
<130> T 0060 WO
<150> EP 08010829.3
   <151> 2008-06-13
<160> 8
<170> PatentIn version 3.3
<210> 1
   <211> 520
   <212> PRT
   <213> Norovirus
<400> 1
   Met Gly Gly Asp Ser Lys Gly Thr Tyr Cys Gly Ala Pro Ile Leu Gly
   1               5                   10                  15
   Pro Gly Ser Ala Pro Lys Leu Ser Thr Lys Thr Lys Phe Trp Arg Ser
               20                  25                  30
   Ser Thr Thr Pro Leu Pro Pro Gly Thr Tyr Glu Pro Ala Tyr Leu Gly
           35                  40                  45
   Gly Lys Asp Pro Arg Val Lys Gly Gly Pro Ser Leu Gln Gln Val Met
       50                  55                  60
   Arg Asp Gln Leu Lys Pro Phe Thr Glu Pro Arg Gly Lys Pro Pro Lys
   65                  70                  75                  80
   Pro Ser Val Leu Glu Ala Ala Lys Lys Thr Ile Ile Asn Val Leu Glu
                   85                  90                  95
   Gln Thr Ile Asp Pro Pro Glu Lys Trp Ser Phe Thr Gln Ala Cys Ala
               100                 105                 110
   Ser Leu Asp Lys Thr Thr Ser Ser Gly His Pro His His Met Arg Lys
           115                 120                 125
   Asn Asp Cys Trp Asn Gly Glu Ser Phe Thr Gly Lys Leu Ala Asp Gln
       130                 135                 140
   Ala Ser Lys Ala Asn Leu Met Phe Glu Gly Gly Lys Asn Met Thr Pro
   145 150 155 160
   Val Tyr Thr Gly Ala Leu Lys Asp Glu Leu Val Lys Thr Asp Lys Ile
   165 170 175
   Tyr Gly Lys Ile Lys Lys Arg Leu Leu Trp Gly Ser Asp Leu Ala Thr
   180 185 190
   Met Ile Arg Cys Ala Arg Ala Phe Gly Gly Leu Met Asp Glu Leu Lys
   195 200 205
   Ala His Cys Val Thr Leu Pro Ile Arg Val Gly Met Asn Met Asn Glu
   210 215 220
   Asp Gly Pro Ile Ile Phe Glu Arg His Ser Arg Tyr Lys Tyr His Tyr
   225 230 235 240
   Asp Ala Asp Tyr Ser Arg Trp Asp Ser Thr Gln Gln Arg Ala Val Leu
   245 250 255
   Ala Ala Ala Leu Glu Ile Met Val Lys Phe Ser Ser Glu Pro His Leu
   260 265 270
   Ala Gln Val Val Ala Glu Asp Leu Leu Ser Pro Ser Val Val Asp Val
   275 280 285
   Gly Asp Phe Lys Ile Ser Ile Asn Glu Gly Leu Pro Ser Gly Val Pro
   290 295 300
   Cys Thr Ser Gln Trp Asn Ser Ile Ala His Trp Leu Leu Thr Leu Cys
   305 310 315 320
   Ala Leu Ser Glu Val Thr Asn Leu Ser Pro Asp Ile Ile Gln Ala Asn
   325 330 335
   Ser Leu Phe Ser Phe Tyr Gly Asp Asp Glu Ile Val Ser Thr Asp Ile
   340 345 350
   Lys Leu Asp Pro Glu Lys Leu Thr Ala Lys Leu Lys Glu Tyr Gly Leu
   355 360 365
   Lys Pro Thr Arg Pro Asp Lys Thr Glu Gly Pro Leu Val Ile Ser Glu
       370                 375                 380
   Asp Leu Asn Gly Leu Thr Phe Leu Arg Arg Thr Val Thr Arg Asp Pro
   385                 390                 395                 400
   Ala Gly Trp Phe Gly Lys Leu Glu Gln Ser Ser Ile Leu Arg Gln Met
                   405                 410                 415
   Tyr Trp Thr Arg Gly Pro Asn His Glu Asp Pro Ser Glu Thr Met Ile
               420                 425                 430
   Pro His Ser Gln Arg Pro Ile Gln Leu Met Ser Leu Leu Gly Glu Ala
           435                 440                 445
   Ala Leu His Gly Pro Ala Phe Tyr Ser Lys Ile Ser Lys Leu Val Ile
       450                 455                 460
   Ala Glu Leu Lys Glu Gly Gly Met Asp Phe Tyr Val Pro Arg Gln Glu
   465                 470                 475                 480
   Pro Met Phe Arg Trp Met Arg Phe Ser Asp Leu Ser Thr Trp Glu Gly
                   485                 490                 495
   Asp Arg Asn Leu Ala Pro Ser Phe Val Asn Glu Asp Gly Val Glu Val
               500                 505                 510
   Asp Lys Leu Ala Ala Ala Leu Glu
           515                 520
<210> 2
   <211> 517
   <212> PRT
   <213> Sapovirus
<400> 2
   Met Lys Asp Glu Phe Gln Trp Lys Gly Leu Pro Val Val Lys Ser Gly
   1               5                   10                  15
   Leu Asp Val Gly Gly Met Pro Thr Gly Thr Arg Tyr His Arg Ser Pro
               20                  25                  30
   Ala Trp Pro Glu Glu Gln Pro Gly Glu Thr His Ala Pro Ala Pro Phe
           35                  40                  45
   Gly Ala Gly Asp Lys Arg Tyr Thr Phe Ser Gln Thr Glu Met Leu Val
       50                  55                  60
   Asn Gly Leu Lys Pro Tyr Thr Glu Pro Thr Ala Gly Val Pro Pro Gln
   65                  70                  75                  80
   Leu Leu Ser Arg Ala Val Thr His Val Arg Ser Tyr Ile Glu Thr Ile
                   85                  90                  95
   Ile Gly Thr His Arg Ser Pro Val Leu Thr Tyr His Gln Ala Cys Glu
               100                 105                 110
   Leu Leu Glu Arg Thr Thr Ser Cys Gly Pro Phe Val Gln Gly Leu Lys
           115                 120                 125
   Gly Asp Tyr Trp Asp Glu Glu Gln Gln Gln Tyr Thr Gly Val Leu Ala
       130                 135                 140
   Asn His Leu Glu Gln Ala Trp Asp Lys Ala Asn Lys Gly Ile Ala Pro
   145                 150                 155                 160
   Arg Asn Ala Tyr Lys Leu Ala Leu Lys Asp Glu Leu Arg Pro Ile Glu
                   165                 170                 175
   Lys Asn Lys Ala Gly Lys Arg Arg Leu Leu Trp Gly Cys Asp Ala Ala
               180                 185                 190
   Thr Thr Leu Ile Ala Thr Ala Ala Phe Lys Ala Val Ala Thr Arg Leu
           195                 200                 205
   Gln Val Val Thr Pro Met Thr Pro Val Ala Val Gly Ile Asn Met Asp
       210                 215                 220
   Ser Val Gln Met Gln Val Met Asn Asp Ser Leu Lys Gly Gly Val Leu
   225                 230                 235                 240
   Tyr Cys Leu Asp Tyr Ser Lys Trp Asp Ser Thr Gln Asn Pro Ala Val
                   245                 250                 255
   Thr Ala Ala Ser Leu Ala Ile Leu Glu Arg Phe Ala Glu Pro His Pro
               260                 265                 270
   Ile Val Ser Cys Ala Ile Glu Ala Leu Ser Ser Pro Ala Glu Gly Tyr
           275                 280                 285
   Val Asn Asp Ile Lys Phe Val Thr Arg Gly Gly Leu Pro Ser Gly Met
       290                 295                 300
   Pro Phe Thr Ser Val Val Asn Ser Ile Asn His Met Ile Tyr Val Ala
   305                 310                 315                 320
   Ala Ala Ile Leu Gln Ala Tyr Glu Ser His Asn Val Pro Tyr Thr Gly
                   325                 330                 335
   Asn Val Phe Gln Val Glu Thr Val His Thr Tyr Gly Asp Asp Cys Met
               340                 345                 350
   Tyr Ser Val Cys Pro Ala Thr Ala Ser Ile Phe His Ala Val Leu Ala
           355                 360                 365
   Asn Leu Thr Ser Tyr Gly Leu Lys Pro Thr Ala Ala Asp Lys Ser Asp
       370                 375                 380
   Ala Ile Lys Pro Thr Asn Thr Pro Val Phe Leu Lys Arg Thr Phe Thr
   385                 390                 395                 400
   Gln Thr Pro His Gly Val Arg Ala Leu Leu Asp Ile Thr Ser Ile Thr
                   405                 410                 415
   Arg Gln Phe Tyr Trp Leu Lys Ala Asn Arg Thr Ser Asp Pro Ser Ser
               420                 425                 430
   Pro Pro Ala Phe Asp Arg Gln Ala Arg Ser Ala Gln Leu Glu Asn Ala
           435                 440                 445
   Leu Ala Tyr Ala Ser Gln His Gly Pro Val Val Phe Asp Thr Val Arg
       450                 455                 460
   Gln Ile Ala Ile Lys Thr Ala Gln Gly Glu Gly Leu Val Leu Val Asn
   465                 470                 475                 480
   Thr Asn Tyr Asp Gln Ala Leu Ala Thr Tyr Asn Ala Trp Phe Ile Gly
                   485                 490                 495
   Gly Thr Val Pro Asp Pro Val Gly His Thr Glu Gly Thr His Lys Ile
               500                 505                 510
   Val Phe Glu Met Glu
           515
<210> 3
   <211> 533
   <212> PRT
   <213> Vesivirus
<400> 3
   Met Lys Val Thr Thr Gln Lys Tyr Asp Val Thr Lys Pro Asp Ile Ser
   1               5                   10                  15
   Tyr Lys Gly Leu Ile Cys Lys Gln Leu Asp Glu Ile Arg Val Ile Pro
               20                  25                  30
   Lys Gly Thr Arg Leu His Val Ser Pro Ala His Thr Asp Asp Tyr Asp
           35                  40                  45
   Glu Cys Ser His Gln Pro Ala Ser Leu Gly Ser Gly Asp Pro Arg Cys
       50                  55                  60
   Pro Lys Ser Leu Thr Ala Ile Val Val Asp Ser Leu Lys Pro Tyr Cys
   65                  70                  75                  80
   Glu Lys Thr Asp Gly Pro Pro His Asp Ile Leu His Arg Val Gln Arg
                   85                  90                  95
   Met Leu Ile Asp His Leu Ser Gly Phe Val Pro Met Asn Ile Ser Ser
               100                 105                 110
   Glu Pro Ser Met Leu Ala Ala Phe His Lys Leu Asn His Asp Thr Ser
           115                 120                 125
   Cys Gly Pro Tyr Leu Gly Gly Arg Lys Lys Asp His Met Ile Gly Gly
       130                 135                 140
   Glu Pro Asp Lys Pro Leu Leu Asp Leu Leu Ser Ser Lys Trp Lys Leu
   145                 150                 155                 160
   Ala Thr Gln Gly Ile Gly Leu Pro His Glu Tyr Thr Ile Gly Leu Lys
                   165                 170                 175
   Asp Glu Leu Arg Pro Val Glu Lys Val Gln Glu Gly Lys Arg Arg Met
   180 185 190
   Ile Trp Gly Cys Asp Val Gly Val Ala Thr Val Cys Ala Ala Ala Phe
   195 200 205
   Lys Gly Val Ser Asp Ala Ile Thr Ala Asn His Gln Tyr Gly Pro Val
   210 215 220
   Gln Val Gly Ile Asn Met Asp Gly Pro Ser Val Glu Ala Leu Tyr Gln
   225 230 235 240
   Arg Ile Arg Ser Ala Ala Lys Val Phe Ala Val Asp Tyr Ser Lys Trp
   245 250 255
   Asp Ser Thr Gln Ser Pro Arg Val Ser Ala Ala Ser Ile Asp Ile Leu
   260 265 270
   Arg Tyr Phe Ser Asp Arg Ser Pro Ile Val Asp Ser Ala Ala Asn Thr
   275 280 285
   Leu Lys Ser Pro Pro Ile Ala Ile Phe Asn Gly Val Ala Val Lys Val
   290 295 300
   Thr Ser Gly Leu Pro Ser Gly Met Pro Leu Thr Ser Val Ile Asn Ser
   305 310 315 320
   Leu Asn His Cys Leu Tyr Val Gly Cys Ala Ile Leu Gln Ser Leu Glu
   325 330 335
   Ser Arg Asn Ile Pro Val Thr Trp Asn Leu Phe Ser Thr Phe Asp Met
   340 345 350
   Met Thr Tyr Gly Asp Asp Gly Val Tyr Met Phe Pro Met Met Phe Ala
   355 360 365
   Ser Val Ser Asp Gln Ile Phe Ala Asn Leu Thr Ala Tyr Gly Leu Lys
   370 375 380
   Pro Thr Arg Val Asp Lys Ser Val Gly Ala Ile Glu Pro Ile Asp Pro
   385 390 395 400
   Glu Ser Val Val Phe Leu Lys Arg Thr Ile Thr Arg Thr Pro His Gly
   405 410 415
   Ile Arg Gly Leu Leu Asp Arg Gly Ser Ile Ile Arg Gln Phe Tyr Tyr
   420 425 430
   Ile Lys Gly Glu Asn Ser Asp Asp Trp Lys Thr Pro Pro Lys Thr Ile
   435 440 445
   Asp Pro Thr Ser Arg Gly Gln Gln Leu Trp Asn Ala Cys Leu Tyr Ala
   450 455 460
   Ser Gln His Gly Pro Glu Phe Tyr Asn Lys Val Tyr Arg Leu Ala Glu
   465 470 475 480
   Lys Ala Val Glu Tyr Glu Glu Leu His Phe Glu Pro Pro Ser Tyr His
   485 490 495
   Ser Ala Leu Glu His Tyr Asn Asn Gln Phe Asn Gly Val Asp Thr Arg
   500 505 510
   Ser Asp Gln Ile Asp Ala Ser Val Met Thr Asp Leu His Cys Asp Val
   515 520 525
   Phe Glu Val Leu Glu
   530
<210> 4
   <211> 526
   <212> PRT
   <213> Artificial
<220>
   <223> Norovirus-RdRp having his tag
<400> 4
   Met Gly Gly Asp Ser Lys Gly Thr Tyr Cys Gly Ala Pro Ile Leu Gly
   1 5 10 15
   Pro Gly Ser Ala Pro Lys Leu Ser Thr Lys Thr Lys Phe Trp Arg Ser
   20 25 30
   Ser Thr Thr Pro Leu Pro Pro Gly Thr Tyr Glu Pro Ala Tyr Leu Gly
   35 40 45
   Gly Lys Asp Pro Arg Val Lys Gly Gly Pro Ser Leu Gln Gln Val Met
       50                  55                  60
   Arg Asp Gln Leu Lys Pro Phe Thr Glu Pro Arg Gly Lys Pro Pro Lys
   65                  70                  75                  80
   Pro Ser Val Leu Glu Ala Ala Lys Lys Thr Ile Ile Asn Val Leu Glu
                   85                  90                  95
   Gln Thr Ile Asp Pro Pro Glu Lys Trp Ser Phe Thr Gln Ala Cys Ala
               100                 105                 110
   Ser Leu Asp Lys Thr Thr Ser Ser Gly His Pro His His Met Arg Lys
           115                 120                 125
   Asn Asp Cys Trp Asn Gly Glu Ser Phe Thr Gly Lys Leu Ala Asp Gln
       130                 135                 140
   Ala Ser Lys Ala Asn Leu Met Phe Glu Gly Gly Lys Asn Met Thr Pro
   145                 150                 155                 160
   Val Tyr Thr Gly Ala Leu Lys Asp Glu Leu Val Lys Thr Asp Lys Ile
                   165                 170                 175
   Tyr Gly Lys Ile Lys Lys Arg Leu Leu Trp Gly Ser Asp Leu Ala Thr
               180                 185                 190
   Met Ile Arg Cys Ala Arg Ala Phe Gly Gly Leu Met Asp Glu Leu Lys
           195                 200                 205
   Ala His Cys Val Thr Leu Pro Ile Arg Val Gly Met Asn Met Asn Glu
       210                 215                 220
   Asp Gly Pro Ile Ile Phe Glu Arg His Ser Arg Tyr Lys Tyr His Tyr
   225                 230                 235                 240
   Asp Ala Asp Tyr Ser Arg Trp Asp Ser Thr Gln Gln Arg Ala Val Leu
                   245                 250                 255
   Ala Ala Ala Leu Glu Ile Met Val Lys Phe Ser Ser Glu Pro His Leu
               260                 265                 270
   Ala Gln Val Val Ala Glu Asp Leu Leu Ser Pro Ser Val Val Asp Val
           275                 280                 285
   Gly Asp Phe Lys Ile Ser Ile Asn Glu Gly Leu Pro Ser Gly Val Pro
       290                 295                 300
   Cys Thr Ser Gln Trp Asn Ser Ile Ala His Trp Leu Leu Thr Leu Cys
   305                 310                 315                 320
   Ala Leu Ser Glu Val Thr Asn Leu Ser Pro Asp Ile Ile Gln Ala Asn
                   325                 330                 335
   Ser Leu Phe Ser Phe Tyr Gly Asp Asp Glu Ile Val Ser Thr Asp Ile
               340                 345                 350
   Lys Leu Asp Pro Glu Lys Leu Thr Ala Lys Leu Lys Glu Tyr Gly Leu
           355                 360                 365
   Lys Pro Thr Arg Pro Asp Lys Thr Glu Gly Pro Leu Val Ile Ser Glu
       370                 375                 380
   Asp Leu Asn Gly Leu Thr Phe Leu Arg Arg Thr Val Thr Arg Asp Pro
   385                 390                 395                 400
   Ala Gly Trp Phe Gly Lys Leu Glu Gln Ser Ser Ile Leu Arg Gln Met
                   405                 410                 415
   Tyr Trp Thr Arg Gly Pro Asn His Glu Asp Pro Ser Glu Thr Met Ile
               420                 425                 430
   Pro His Ser Gln Arg Pro Ile Gln Leu Met Ser Leu Leu Gly Glu Ala
           435                 440                 445
   Ala Leu His Gly Pro Ala Phe Tyr Ser Lys Ile Ser Lys Leu Val Ile
       450                 455                 460
   Ala Glu Leu Lys Glu Gly Gly Met Asp Phe Tyr Val Pro Arg Gln Glu
   465                 470                 475                 480
   Pro Met Phe Arg Trp Met Arg Phe Ser Asp Leu Ser Thr Trp Glu Gly
                   485                 490                 495
   Asp Arg Asn Leu Ala Pro Ser Phe Val Asn Glu Asp Gly Val Glu Val
   500 505 510
   Asp Lys Leu Ala Ala Ala Leu Glu His His His His His His
   515 520 525
<210> 5
   <211> 523
   <212> PRT
   <213> Artificial
<220>
   <223> Sapovirus-RdRp having his tag
<400> 5
   Met Lys Asp Glu Phe Gln Trp Lys Gly Leu Pro Val Val Lys Ser Gly
   1               5                   10                  15
   Leu Asp Val Gly Gly Met Pro Thr Gly Thr Arg Tyr His Arg Ser Pro
               20                  25                  30
   Ala Trp Pro Glu Glu Gln Pro Gly Glu Thr His Ala Pro Ala Pro Phe
           35                  40                  45
   Gly Ala Gly Asp Lys Arg Tyr Thr Phe Ser Gln Thr Glu Met Leu Val
       50                  55                  60
   Asn Gly Leu Lys Pro Tyr Thr Glu Pro Thr Ala Gly Val Pro Pro Gln
   65                  70                  75                  80
   Leu Leu Ser Arg Ala Val Thr His Val Arg Ser Tyr Ile Glu Thr Ile
                   85                  90                  95
   Ile Gly Thr His Arg Ser Pro Val Leu Thr Tyr His Gln Ala Cys Glu
               100                 105                 110
   Leu Leu Glu Arg Thr Thr Ser Cys Gly Pro Phe Val Gln Gly Leu Lys
           115                 120                 125
   Gly Asp Tyr Trp Asp Glu Glu Gln Gln Gln Tyr Thr Gly Val Leu Ala
       130                 135                 140
   Asn His Leu Glu Gln Ala Trp Asp Lys Ala Asn Lys Gly Ile Ala Pro
   145                 150                 155                 160
   Arg Asn Ala Tyr Lys Leu Ala Leu Lys Asp Glu Leu Arg Pro Ile Glu
   165 170 175
   Lys Asn Lys Ala Gly Lys Arg Arg Leu Leu Trp Gly Cys Asp Ala Ala
   180 185 190
   Thr Thr Leu Ile Ala Thr Ala Ala Phe Lys Ala Val Ala Thr Arg Leu
   195 200 205
   Gln Val Val Thr Pro Met Thr Pro Val Ala Val Gly Ile Asn Met Asp
   210 215 220
   Ser Val Gln Met Gln Val Met Asn Asp Ser Leu Lys Gly Gly Val Leu
   225 230 235 240
   Tyr Cys Leu Asp Tyr Ser Lys Trp Asp Ser Thr Gln Asn Pro Ala Val
   245 250 255
   Thr Ala Ala Ser Leu Ala Ile Leu Glu Arg Phe Ala Glu Pro His Pro
   260 265 270
   Ile Val Ser Cys Ala Ile Glu Ala Leu Ser Ser Pro Ala Glu Gly Tyr
   275 280 285
   Val Asn Asp Ile Lys Phe Val Thr Arg Gly Gly Leu Pro Ser Gly Met
   290 295 300
   Pro Phe Thr Ser Val Val Asn Ser Ile Asn His Met Ile Tyr Val Ala
   305 310 315 320
   Ala Ala Ile Leu Gln Ala Tyr Glu Ser His Asn Val Pro Tyr Thr Gly
   325 330 335
   Asn Val Phe Gln Val Glu Thr Val His Thr Tyr Gly Asp Asp Cys Met
   340 345 350
   Tyr Ser Val Cys Pro Ala Thr Ala Ser Ile Phe His Ala Val Leu Ala
   355 360 365
   Asn Leu Thr Ser Tyr Gly Leu Lys Pro Thr Ala Ala Asp Lys Ser Asp
   370 375 380
   Ala Ile Lys Pro Thr Asn Thr Pro Val Phe Leu Lys Arg Thr Phe Thr
   385 390 395 400
   Gln Thr Pro His Gly Val Arg Ala Leu Leu Asp Ile Thr Ser Ile Thr
   405 410 415
   Arg Gln Phe Tyr Trp Leu Lys Ala Asn Arg Thr Ser Asp Pro Ser Ser
   420 425 430
   Pro Pro Ala Phe Asp Arg Gln Ala Arg Ser Ala Gln Leu Glu Asn Ala
   435 440 445
   Leu Ala Tyr Ala Ser Gln His Gly Pro Val Val Phe Asp Thr Val Arg
   450 455 460
   Gln Ile Ala Ile Lys Thr Ala Gln Gly Glu Gly Leu Val Leu Val Asn
   465 470 475 480
   Thr Asn Tyr Asp Gln Ala Leu Ala Thr Tyr Asn Ala Trp Phe Ile Gly
   485 490 495
   Gly Thr Val Pro Asp Pro Val Gly His Thr Glu Gly Thr His Lys Ile
   500 505 510
   Val Phe Glu Met Glu His His His His His His
   515 520
<210> 6
   <211> 539
   <212> PRT
   <213> Artificial
<220>
   <223> Vesivirus-RdRp having his tag
<400> 6
   Met Lys Val Thr Thr Gln Lys Tyr Asp Val Thr Lys Pro Asp Ile Ser
   1 5 10 15
   Tyr Lys Gly Leu Ile Cys Lys Gln Leu Asp Glu Ile Arg Val Ile Pro
   20 25 30
   Lys Gly Thr Arg Leu His Val Ser Pro Ala His Thr Asp Asp Tyr Asp
   35 40 45
   Glu Cys Ser His Gln Pro Ala Ser Leu Gly Ser Gly Asp Pro Arg Cys
        50                  55                  60
   Pro Lys Ser Leu Thr Ala Ile Val Val Asp Ser Leu Lys Pro Tyr Cys
   65                  70                  75                  80
   Glu Lys Thr Asp Gly Pro Pro His Asp Ile Leu His Arg Val Gln Arg
                   85                  90                  95
   Met Leu Ile Asp His Leu Ser Gly Phe Val Pro Met Asn Ile Ser Ser
               100                 105                 110
   Glu Pro Ser Met Leu Ala Ala Phe His Lys Leu Asn His Asp Thr Ser
           115                 120                 125
   Cys Gly Pro Tyr Leu Gly Gly Arg Lys Lys Asp His Met Ile Gly Gly
       130                 135                 140
   Glu Pro Asp Lys Pro Leu Leu Asp Leu Leu Ser Ser Lys Trp Lys Leu
   145                 150                 155                 160
   Ala Thr Gln Gly Ile Gly Leu Pro His Glu Tyr Thr Ile Gly Leu Lys
                   165                 170                 175
   Asp Glu Leu Arg Pro Val Glu Lys Val Gln Glu Gly Lys Arg Arg Met
               180                 185                 190
   Ile Trp Gly Cys Asp Val Gly Val Ala Thr Val Cys Ala Ala Ala Phe
           195                 200                 205
   Lys Gly Val Ser Asp Ala Ile Thr Ala Asn His Gln Tyr Gly Pro Val
       210                 215                 220
   Gln Val Gly Ile Asn Met Asp Gly Pro Ser Val Glu Ala Leu Tyr Gln
   225                 230                 235                 240
   Arg Ile Arg Ser Ala Ala Lys Val Phe Ala Val Asp Tyr Ser Lys Trp
                   245                 250                 255
   Asp Ser Thr Gln Ser Pro Arg Val Ser Ala Ala Ser Ile Asp Ile Leu
               260                 265                 270
   Arg Tyr Phe Ser Asp Arg Ser Pro Ile Val Asp Ser Ala Ala Asn Thr
   275 280 285
   Leu Lys Ser Pro Pro Ile Ala Ile Phe Asn Gly Val Ala Val Lys Val
   290 295 300
   Thr Ser Gly Leu Pro Ser Gly Met Pro Leu Thr Ser Val Ile Asn Ser
   305 310 315 320
   Leu Asn His Cys Leu Tyr Val Gly Cys Ala Ile Leu Gln Ser Leu Glu
   325 330 335
   Ser Arg Asn Ile Pro Val Thr Trp Asn Leu Phe Ser Thr Phe Asp Met
   340 345 350
   Met Thr Tyr Gly Asp Asp Gly Val Tyr Met Phe Pro Met Met Phe Ala
   355 360 365
   Ser Val Ser Asp Gln Ile Phe Ala Asn Leu Thr Ala Tyr Gly Leu Lys
   370 375 380
   Pro Thr Arg Val Asp Lys Ser Val Gly Ala Ile Glu Pro Ile Asp Pro
   385 390 395 400
   Glu Ser Val Val Phe Leu Lys Arg Thr Ile Thr Arg Thr Pro His Gly
   405 410 415
   Ile Arg Gly Leu Leu Asp Arg Gly Ser Ile Ile Arg Gln Phe Tyr Tyr
   420 425 430
   Ile Lys Gly Glu Asn Ser Asp Asp Trp Lys Thr Pro Pro Lys Thr Ile
   435 440 445
   Asp Pro Thr Ser Arg Gly Gln Gln Leu Trp Asn Ala Cys Leu Tyr Ala
   450 455 460
   Ser Gln His Gly Pro Glu Phe Tyr Asn Lys Val Tyr Arg Leu Ala Glu
   465 470 475 480
   Lys Ala Val Glu Tyr Glu Glu Leu His Phe Glu Pro Pro Ser Tyr His
   485 490 495
   Ser Ala Leu Glu His Tyr Asn Asn Gln Phe Asn Gly Val Asp Thr Arg
                       500                 505                 510
   Ser Asp Gln Ile Asp Ala Ser Val Met Thr Asp Leu His Cys Asp Val
           515                 520                 525
   Phe Glu Val Leu Glu His His His His His His
       530                 535
<210> 7
   <211> 24
   <212> RNA
   <213> Artificial
<220>
   <223> single stranded RNA template strand ssRX21 for
<400> 7
   gcugaugccg ucaaguuuac cccc
   24
<210> 8
   <211> 24
   <212> RNA
   <213> Artificial
<220>
   <223> single stranded antisense product strand ssRX21 rev
<220>
   <221> modified_base
   <222> (10) .. (10)
   <223> cm
<220>
   <221> modified_base
   <222> (15)..(15)
   <223> cm
<220>
   <221> modified_base
   <222> (18)..(18)
   <223> cm
<220>
   <221> modified_base
   <222> (21)..(21)
   <223> cm
<220>
   <221> modified_base
   <222> (24)..(24)
   <223> cm
<400> 8
   ggggguaaac uugacggcau cagc
   24

## Description

The present invention relates to a method for enzymatically synthesizing chemically modified RNA by using RNA-dependent RNA polymerases (RdRp) as defined in claim 1, especially RdRps from viruses of the *Caliciviridae* family. The method of the present invention is particularly useful for preparing RNA molecules of increased stability especially with respect to RNA degradation, for example for *in vivo* applications. Further subject matter of the present invention relates to a kit for carrying out the enzymatic synthesis of the chemically modified RNA.

RNA molecules containing chemically modified ribonucleotides are useful for various applications in science and medicine. In particular, it is generally known that especially backbone-modified RNAs possess an increased stability, e.g. with respect to degradation by RNases, which is of importance for *in vivo* applications, for example in the case of antisense ribonucleotides as well as siRNAs (small interfering RNAs) for RNA interference (RNAi).

Hitherto, such modified RNA is generally prepared by solid-phase chemical synthesis methods. However, such chemical methods have several drawbacks, for example they are expensive and time consuming. Those drawbacks are an important limitation for the broad diffusion of the RNAi-therapy. Indeed, it is predicted that several tons of RNAi-molecules may be needed each year for the treatment of so far untreatable diseases such as cancer, viral infectious diseases and degenerative diseases (i.e. acute macular degeneration). Those so far untreatable diseases may extensively benefit from RNAi-Therapy.

Accordingly, there is a substantial need for alternative methods for the production of chemically modified RNA molecules, in particular for therapeutic applications.

It is known from WO-A-2007/012329 that viruses of the *Caliciviridae* family can be used for amplification and/or labelling of RNA molecules. In this disclosure, the authors also describe that the calicivirus RdRps can make use of fluorescently coupled, biotin-coupled or radioactively-coupled NTPs used for labelling of the RNA. However, concrete examples that the calicivirus RdRps can factually incorporate such coupled NTPs are completely missing. Furthermore, it is clear that the term NTP analogue in WO-A-2007/012329 is exclusively directed to such analogues that can be used for the detection of the labelled RNA by fluorescence emission, radioactive emission and/or by providing a partner for a chemical or biological binding pair such as for example biotin. Thus, the incorporation of NTP-analogues as envisaged in WO-A-2007/012329 is exclusively directed to the detection of coupled NTPs used for detection of RNA molecules labelled with such NTP-analogues.

Furthermore, another group suggested recently the use of chemically modified ribonucleotides for the inhibition of a norovirus RdRp (Zamyatkin et al. (2008) J. Biol. Chem. 283: 7705-7712).

The RdRp-activity of hepatitis-C-virus RdRp and the possibility to use 2'-C-methyl-CTP, 2'-O-methyl-CTP and natural CTP as substrate is analysed in Dutartre et al. (2006) Antimicrob Agents Chemother. 50(12):4161-9.

Duratre et al. (2005) J. Biol. Chem. 280(8):6359-68 describe, that 2'-methyl-GTP works as an inhibitor for the activity of the hepatitis-C-virus RdRp.

WO 01/46936 A discloses an RdRp isolated from double-stranded-RNA-viruses, e.g. Cystoviridae, Reoviridae, Birnaviridae or Totiviridae capable to catalyze RNA synthesis using ssRNA, dsRNA, ssDNA or dsDNA templates.

WO 00/66605 A discloses a process for producing a polynucleotide comprising mRNA, rRNA or viral RNA, whereas more than 25 % of the ribose rings of the polynucleotide are covalently modified at the 2'-OH position. Different nucleic acid polymerases, inter alia RdRps are suggested to be used in this process.

WO 03/038042 A2 describes methods for detecting the presence of a target molecule by generating multiple oligonucleotides through reiterative enzymatic oligonucleotide synthesis events using a polymerase e.g. an RdRp.

Belliot et al. (2004) J Vir 79 (4): 2393-2404, analyzed the effects of several UTP analogs on the RdRp activity of the Norovirus and feline Calicivirus Proteinase-Polymerase, an active form of RdRp.

The solution to this technical problem is provided by the embodiments of the present invention as defined in claims 1 and 20.

In particular, the present invention provides a method for enzymatically synthesizing chemically modified RNA comprising the steps of:
(a) providing ssRNA template (single-stranded RNA template); and
(b) interaction of the ssRNA template with a protein having RNA-dependent RNA polymerase (RdRp) activity under conditions sufficient for RNA synthesis in the presence of at least one ribonucleotide triphosphate having chemical modification at the ribose, phosphate and/or base moiety to provide chemically modified double-stranded RNA (dsRNA), wherein the chemical modification does not result in labelling of the dsRNA with a radioactive, fluorescent and/or chemical label used for detection of said dsRNA,
wherein the protein having RdRp activity is as defined in claim 1.

In view of the prior art it is highly surprising that RdRps, especially corresponding enzymes from the calicivirus family (*Caliciviridae*) can factually incorporate diverse chemically modified ribonucleotides into dsRNA by synthesizing a complementary strand to a single stranded RNA (ssRNA) template.

It is further contemplated according to the present invention that the incorporation of chemically modified ribonucleoside triphosphates into RNA will be used for the large-scale industrial production of such modified RNA. Thus, it is preferred that chemically modified ribonucleoside triphosphates are used in an amplification reaction which comprises the further steps:
(c) separating the dsRNA into ssRNA, and optionally performing the following steps (d) to (f):
(d) repeating steps (b) and (c) n times with n being an integer of at least 1;
(e) carrying out a final step (b); and
(f) stopping the reaction.

Preferably, n is an integer from 10 to 50, more preferred 15 to 40, most preferred 20 to 30.

The strand separation according step (c) may be carried out by application of heat (heat denaturation), by chemical denaturation or enzymatically, preferably by a helicase. Especially in the case of heat denaturation, it is desirable to add further RdRp molecules to the reaction mixture for performing the next step (b).

According to the present invention the protein having RdRp activity has a "right hand conformation" and that the amino acid sequence of said protein comprises a conserved arrangement of the following sequence motifs:
a. XXDYS
b. GXPSG
c. YGDD
d. XXYGL
e. XXXXFLXRXX
with the following meanings:
D: aspartate
Y: tyrosine
S: serine
G: glycine
P: proline
L: leucine
F: phenylalanine
R: arginine
X: any amino acid.

The so-called "right hand conformation" as used herein means that the tertiary structure (conformation) of the protein folds like a right hand with finger, palm and thumb, as observed in most template-dependent polymerases

The sequence motif "XXDYS" is the so-called A-motif. The A-motif is responsible for the discrimination between ribonucleosides and deoxyribonucleosides. The motif "GXPSG" is the so-called B-motif. The B-motif is conserved within all representatives of this RdRp family of the corresponding polymerases from the *Caliciviridae.* The motif "YGDD" ("C-motif) represents the active side of the enzyme. This motif, in particular the first aspartate residue (in bold, YGDD) plays an important role in the coordination of the metal ions during the Mg²⁺/Mn²⁺ dependent catalysis. The motif "XXYGL" is the so-called D-motif. The D-motif is a feature of template-dependent polymerases. Finally, the "XXXXFLXRXX" motif (E-motif) is a feature of RNA-dependent RNA polymerases which discriminates them from DNA-dependent RNA polymerases.

Typical representatives of RdRps according to the present invention are the corresponding enzymes of the calicivirus family (*Caliciviridae*)*.* The RdRps of the calicivirus family are capable of synthesizing complementary strands using as a template any ssRNA template *in vitro,* including heterologous viral, eukaryotic and prokaryotic templates. The ssRNA template may be positive stranded or negative stranded.

The above-defined protein having RdRp activity is capable of synthesizing a complementary on a strand both by elongation of a corresponding primer and by novo synthesis of a complementary strand in the absence of a primer. The primer, if desired, may be a sequence specific primer or may be a random primer or may be an oligo-T-primer or an oligo-U-Primer. More details of the characteristic features of the calicivirus RdRp can be found in WO-A-2007/012329.

Preferably, the RNA-dependent RNA-polymerase is an RdRp of a human and/or non-human pathogenic calicivirus. Especially preferred is an RdRp of a norovirus, sapovirus, vesivirus or lagovirus, for example the RdRP of the norovirus strain HuCV/NL/Dresden174/1997/GE (GenBank acc. No AY741811) or of the sapovirus strain pJG-Sap01 (GenBank acc. No AY694184) or an RNA-dependent RNA polymerase of the vesivirus strain FCV/Dresden/2006/GE (GeBank acc. No DQ424892).

According to especially preferred embodiments of the invention the RdRp is a protein having an amino acid sequence according SEQ ID NO: 1 (norovirus-RdRP), SEQ ID NO: 2 (sapovirus-RdRP) or SEQ ID NO: 3 (vesivirus-RdRP). The person skilled in the art is readily capable of preparing such RdRP, for example by recombinant expression using suitable expression vectors and host organisms (cf. WO-A-2007/012329). To facilitate purification of the RdRp in recombinant expression, it is preferred that the RdRp is expressed with a suitable tag (for example GST or (His)₆-tag) at the N- or C-terminus of the corresponding sequence. For example, a histidine tag allows the purification of the protein by affinity chromatography over a nickel or cobalt column in a known fashion. Examples of embodiments of RdRps fused to a histidine tag are the proteins having an amino acid sequence according to SEQ ID NO: 4, SEQ ID NO: 5 or SEQ ID NO: 6. SEQ ID NO: 4 corresponds to a norovirus-RdRP having a histidine tag. SEQ ID NO: 5 corresponds to the amino acid sequence of a sapovirus-RdRP having a histidine tag. SEQ ID NO: 6 corresponds to the amino acid sequence of vesirius-RdRP having a histidine tag.
SEQ ID NO: 1:
SEQ ID NO: 2:
SEQ ID NO: 3:
SEQ ID NO: 4:
SEQ ID NO: 5:
SEQ ID NO: 6:

The method of the present invention is particularly useful for providing short RNA molecules for gene silencing applications, either by antisense technology or RNA interference.

Therefore, the ssRNA template to be used in the method of the present invention has preferably a length of 8 to 45 nucleotides, preferably of 15 to 30 nucleotides, preferably of 21 to 28 nucleotides, more preferably of 21 to 23 nucleotides. The molecules of the latter length are particularly useful for siRNA applications. The chemically modified RNA products of the methods of the present invention preferably have an increased stability as compared to the non-modified dsRNA analogues.

Especially for this purpose, the chemical modification of the at least one modified ribonucleoside triphosphate to be incorporated by the RdRp activity into the complementary strand can have a chemical modification(s) at the ribose, phosphate and/or base moiety. With respect to molecules having an increased stability, especially with respect to RNA degrading enzymes, modifications at the backbone, i.e. the ribose and/or phosphate moieties, are especially preferred.

Preferred examples of ribose-modified ribonucleoside triphosphates are analogues wherein the 2'-OH group is replaced by a group selected from H, OR, R, halo, SH, SR, NH₂, NHR, NR₂ or CN with R being C₁-C₆ alkyl, alkenyl or alkynyl and halo being F, Cl, Br or I. It is clear in the context of the present invention, that the term "modified ribonucleoside triphosphate" or "modified ribonucleotide" also includes 2'-deoxy derivatives which may at several instances also be termed "deoxynucleotides".

Typical examples of such ribonucleotide analogues with a modified ribose at the 2' position include 2'-O-methyl-cytidine-5'-triphosphate, 2'-amino-2'-deoxy-uridine, 2'-azido-2'-deoxy-uridine-5'-triphosphate, 2'-fluoro-2'-deoxy-guanosine-5'-triphosphate and 2'-O-methyl-5-methyl-uridine-5'-triphosphate.

Examples of ribonucleoside triphosphates leading to a phosphate backbone modification in the desired dsRNA product are phosphothioate analogues.

According to the present invention, the at least one modified ribonucleoside trisphophate may also be selected from analogues having a chemical modification at the base moiety. Examples of such analogues include 5-aminoallyl-uridine-5'-triphosphate, 6-aza-uridine-5'-triphosphate, 8-aza-adenosine-5'-triphosphate, 5-bromo-uridine-5-'triphosphate, 7-deaza-adenosine-5'-triphosphate, 7-deaza-guanosine-5'-triphosphate, N⁶-methyl-adenosine-5'-triphosphate, 5-methyl- cytidine-5-'triphosphate, pseudo-uridine-5'-triphosphate, and 4-thio-uridine-5'-triphosphate.

The above and other chemically modified ribonucleoside triphosphates are commercially available, for example from Sigma-Aldrich Chemie GmbH, Munich, Germany or Trilink technologies, USA.

According to the present invention, the term "conditions sufficient for RNA synthesis" means the conditions, in particular relating to buffer, temperature, salt and metal ion (if applicable) conditions that allow the RdRp to synthesise an RNA strand complementary to a template strand. Appropriate buffer, salt, metal ion, reducing agent (if applicable) and other conditions of RdRps are known to the skilled person. With regard to the RdRPs of caliciviruses, it is referred to WO-A-2007/012329. Thus, the ssRNA template is used in amounts of, e.g. 1 microgram to 4 microgram per 50 microliter reaction volume. The concentration of the ribonucleoside triphosphates (including the modified ribonucleoside trisphosphate (s)) is preferably in the range of from 0.1 micromol/l to 1 micromol/l, for example 0.4 micromol/l. The concentration of the RdRp may be for example 1 micromol/l to 10 micromol/l.

Typical buffer conditions are 10 to 80 mM, more preferred 20 to 50 mM HEPES, pH 7.0-8.0, 1 to 5 mM, for example 3 mM magnesium acetate, magnesium chloride, manganese acetate or manganese chloride and 1 to 5 mM of a reducing agent, for example DTT.

A typical stop solution contains 2 to 10 mM, preferably 4 to 8 mM ammonium acetate, and 50 to 200 mM, for example 150 mM EDTA.

Short RNA templates (e.g. as defined above) are usually prepared by chemical synthesis of two separate strands that are then annealed in vitro using an annealing buffer containing for example Tris-Hcl 50 mM, pH 8.0, NaCl 200 mM, EDTA 1mM. Other methods for providing the ssRNA template include enzymatic manipulations, for example transcription initiation depending on selected sequences (so called "promotor") or using a specific primer and RNA synthesis by DNA-dependent RNA polymerases following degradation of the DNA strand.

Preferred reaction volumes range from 20 to 200 microliter, preferably 50 to 100 microliter. Typically, the buffer conditions and other conditions as outlined above are provided by mixing appropriate stock solutions (usually 5x or 10x concentrated), adding the RdRP and the ssRNA template and double distilled or deionised water to the desired final reaction volume.

A further subject matter of the present invention is a kit for carrying out the inventive method. The kit of the present invention comprises a protein having RdRp activity (preferred examples are defined above), ribonucleotides (rATP, rGTP, rCTP, rUTP, preferably as stock solutions), at least one chemically modified ribonucleoside triphosphate (as defined above and preferably selected from the examples outlined above), an appropriate buffer (preferably as a stock solution as defined above), a helicase and, optionally, a stop solution (preferably a stop solution as defined above, more preferred in the form of a 5x or 10x stock solution).

The figures show:

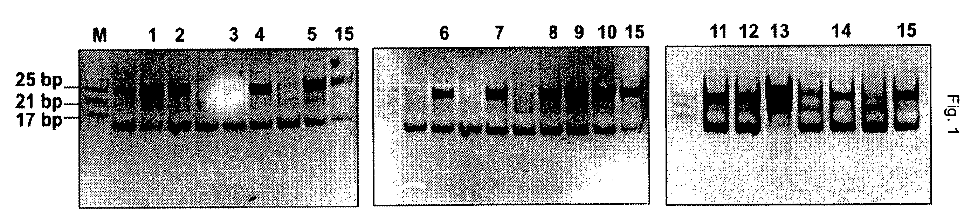

- Fig. 1: shows photographic representations of polyacrylamid gel electrophoretic analysis after ethidium bromide staining of double-stranded RNA with or without modified nucleotides synthesized using calicivirus RdRp. M, molecular size marker for double-stranded RNA (Ambion/Applied Biosystems, Foster City, CA, USA) is indicated. Single-stranded RNA template is the band below the 17 bp band of the marker M. The dsRNA product synthesized by the Calicivirus RdRp is visible at around 25 bp band of the marker M.

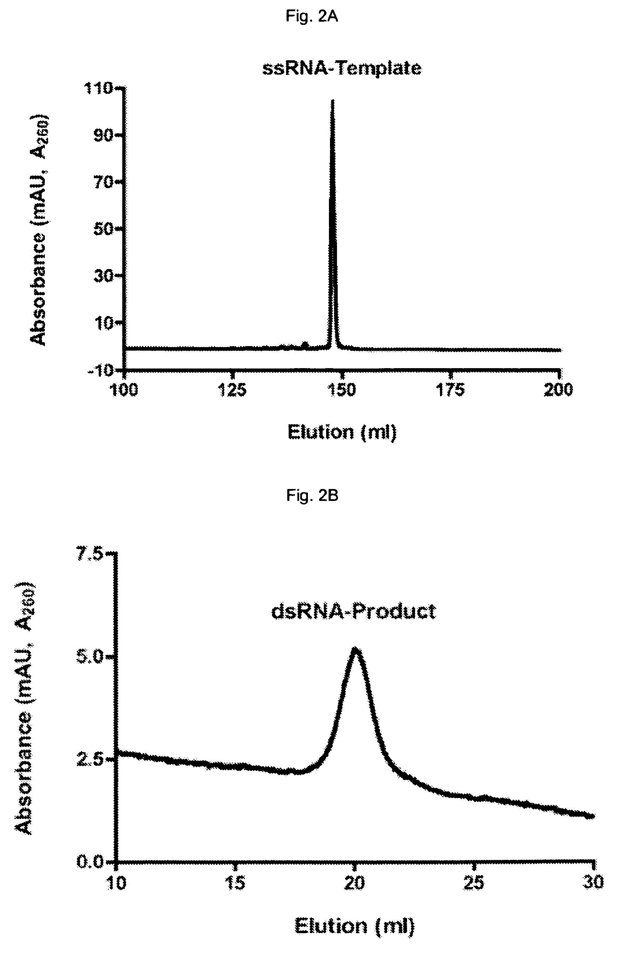

- Fig. 2a: shows graphical representations of elution profiles resulting in purification of the single-stranded RNA template used for enzymatic synthesis with calicivirus RdRp.
- Fig. 2b: shows graphical representations of elution profiles resulting in purification of the double-stranded RNA products of the enzymatic synthesis using calicivirus RdRp. The product of a reaction for a template ssRNA was purified from the reaction mixture by ion exchange chromatography and fractionized.

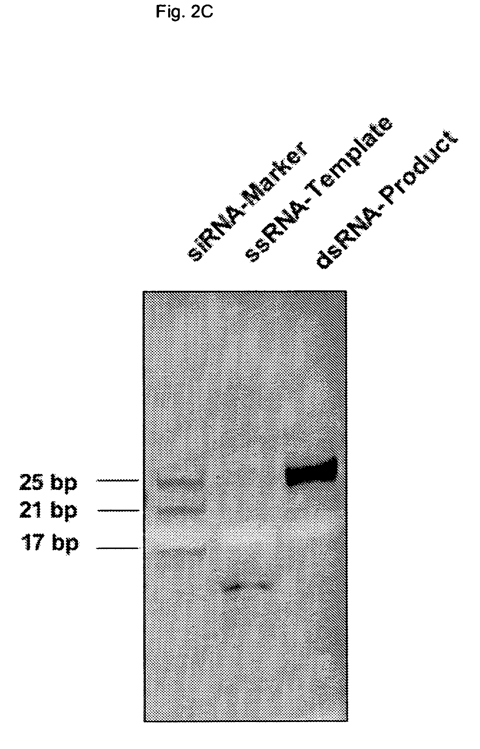

- Fig. 2c: shows a photographical representation of a polyacrylamid gel electrophoresis of the fractions indicated (marked with a dashed box) in Fig. 2b. M, molecular size marker for double-stranded RNA (Ambion/Applied Biosystem) as indicated. The template ssRNA migrates with the 17 bp marker fragment. The product of the synthesis by the calicivirus RdRp can also be seen as a band of approximately 25 bp.

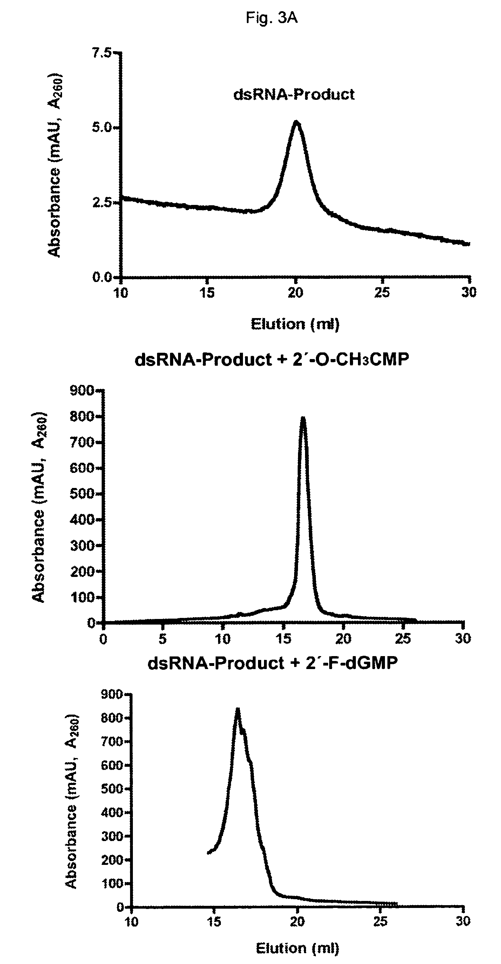

- Fig. 3a: shows graphical representations of elution profiles resulting in purification of the double-stranded RNA products of the enzymatic synthesis using calicivirus RdRp. The products of a reaction for a normal dsRNA product (upper panel), a dsRNA product including 2-O-CH3-CMP (2'-O-methyl-cytidine-5'-monophosphate, medium panel) and a dsRNA containing 2-F-dGMP (2'-fluoro-2'-deoxy- guanosine-5'-monophosphate) were purified from the reaction mixture by ion exchange chromatography and fractionized.

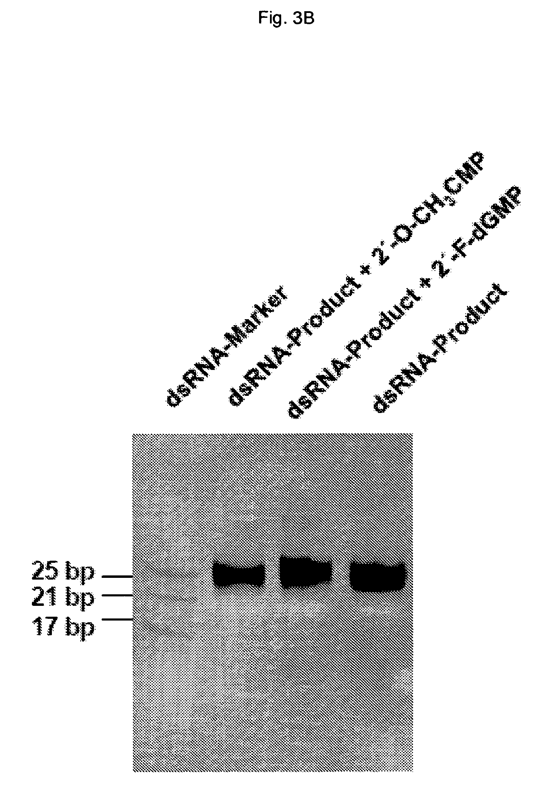

- Fig. 3b: shows a photographical representation of a polyacrylamid gel electrophoresis of the fractions indicated (marked with a dashed box) in Fig. 3a. M, molecular size marker for double-stranded RNA (Ambion/Applied Biosystem) as indicated. The product of the synthesis using unmodified nucleotides (dsRNA-product) co-migrates with the 25 bp marker fragment. The product of the synthesis with incorporation of 2'-O-methyl-cydidine-5'-triphosphate can also be seen as a band of approximately 25 bp. The same holds true for the product of the synthesis under incorporation of 2'-fluoro-2'-deoxy-guanosine-5'-triphosphate running also as a dsRNA product of around 25 bp.

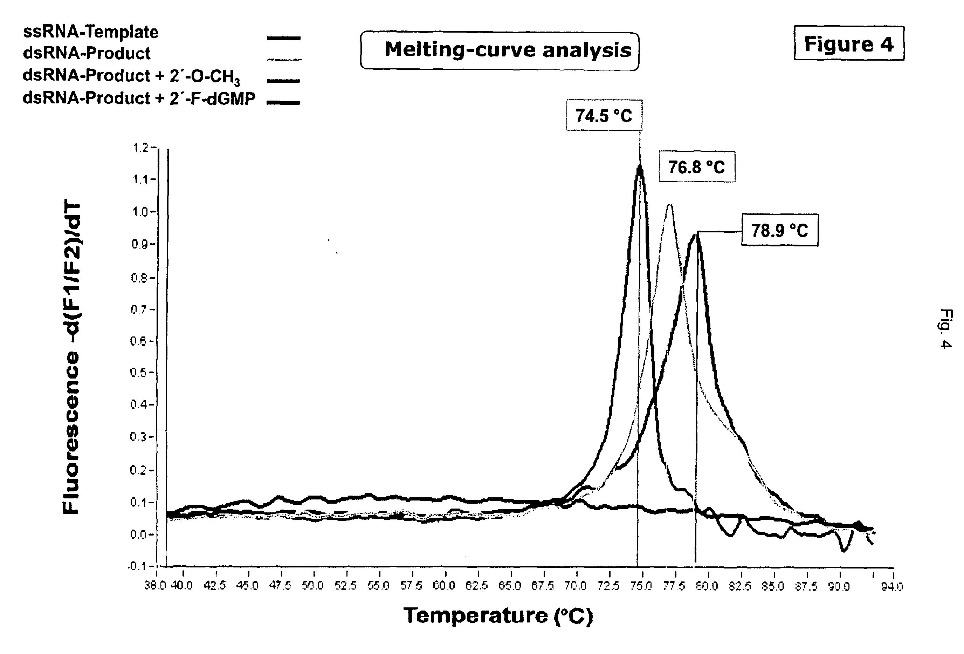

- Fig. 4: is a graph showing results of melting point analyses of the products of the enzymatic synthesis by calicivirus RdRp in the absence or presence of modified ribonucleoside triphosphates. The reaction products of a synthesis using unmodified ribonucleotides (dsRNA product), using 2'-O-CH3-CTP (dsRNA product + 2-O-CH3-CMP) and using 2'-F-dGTP (dsRNA product + 2'F-dGMP) were incubated with SYBR Green I, and the melting points of the products were determined using the LightCycler (Roche diagnostics). The products display different melting points caused by the incorporation of modified nucleotides as compared to unmodified nucleotides.

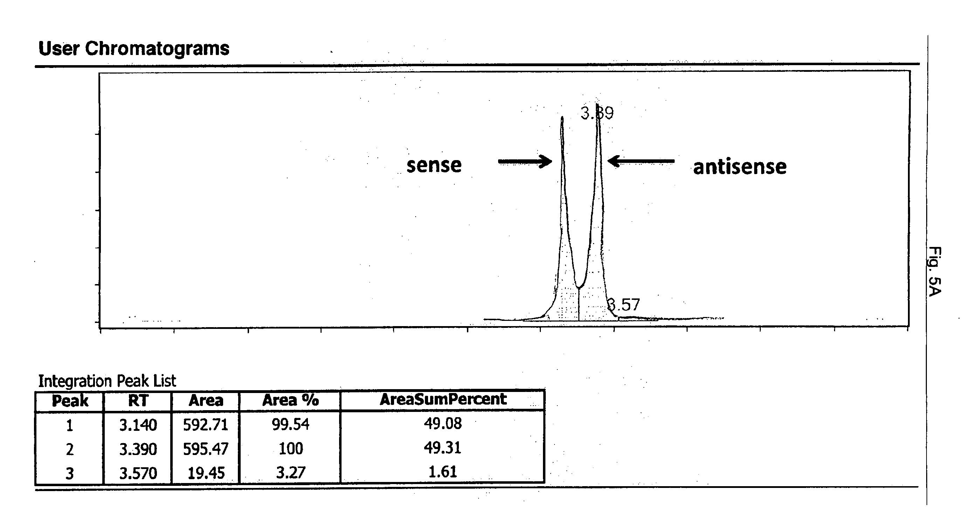

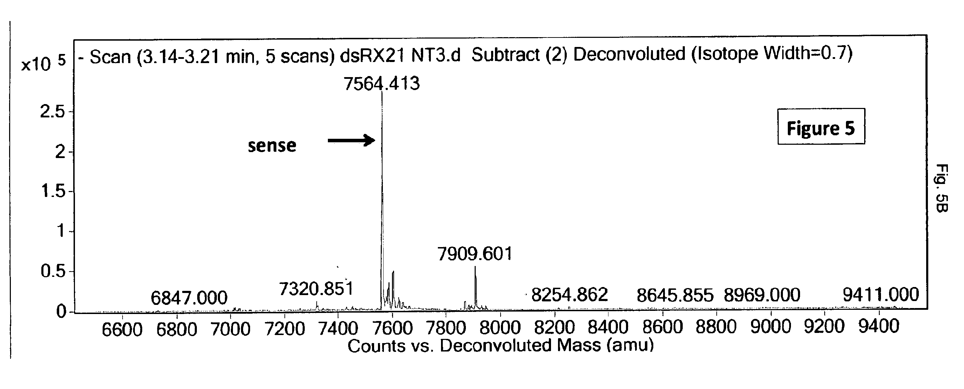

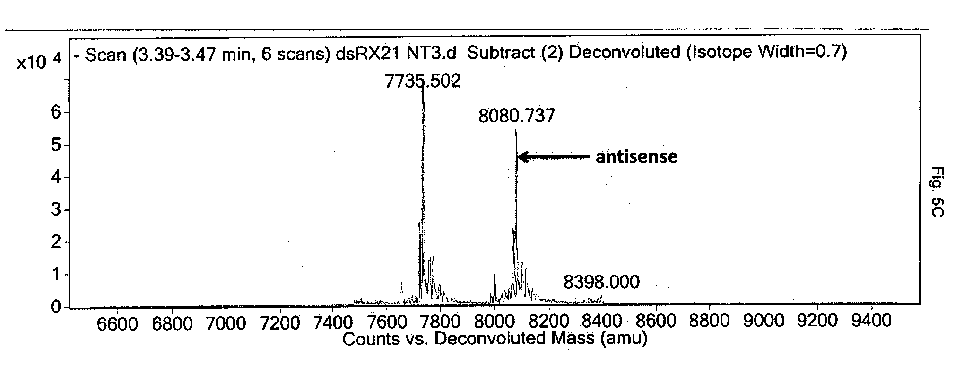

- Fig. 5.: shows the mass spectrometric analysis of HPLC fractions containing the products of enzymatic synthesis using 2'-O-CH3-CTP (dsRNA product + 2-O-CH3-CMP). The masses of the sense strand (template ssRNA, molecular weight of 7564.41) and the antisense strand (synthesized single stranded RNA complementary to the template ssRNA, molecular weight of 8080.73) corrrespond to the predicted incorporation of 2'-O-CH3-CMP in the antisense strand. A: Graphical representation of the elution profile of the HPLC fractions containing the sense and antisense strand (peaks indicated). B: Graphical representation of the mass spectrometric analysis of the sense strand fraction. C: Graphical representation of the mass spectrometric analysis of the antisense strand fraction.

The following non-limiting examples further illustrate the present invention.

### EXAMPLES

### General protocol for enzymatic synthesis of modified RNA

The general reaction mixture is as follows:
The reaction mix consists of
   - RNA-dependent RNA polymerase (RdRp) from calicivurs
   - Buffer (5-fold): HEPES pH 7.6, 250 mM; MnCl2, 25 mM; DTT 5 mM
   - Ribonucleoside triphosphates: X, Y, Z, W
   - Single-stranded RNA (template);
      Sequence: GCUGAUGCCGUCAAGUUUACCCCC (SEQ ID NO: 7)
   - Aqua. Dest

The reaction mixture is incubated for 2 h at 30 to 42°C depending on the RdRp used.

X, Y, Z, or W corresponds to one or more of the following ribonucleoside triphosphates or deoxyribonulcleoside triphosphates alone or in combination:

**Table 1: unmodified and modified nucleotides for X, Y, Z, W**

| |
|---|
| adenosine-5'-triphosphate |
| cytidine-5-'triphosphate |
| guanosine-5'-triphosphate |
| uridine-5'-triphosphate |
| 2'-O-methyl-cytidine-5'-triphosphate |
| 5-aminoallyl-uridine-5'-triphosphate |
| 6-aza-uridine-5'-triphosphate |
| 8-aza-adenosine-5'-triphosphate |
| 5-bromo-uridine-5'-triphosphate |
| 7-deaza-adenosine-5'-triphosphate |
| 7-deaza-guanosine-5'-triphosphate |
| N⁶-methyl-adenosine-5'-triphosphate |
| 5-methyl-cytidine-5-'triphosphate |
| pseudo- uridine-5'-triphosphate |
| 4-thio-uridine-5'-triphosphate |
| 2'-amino-2'-deoxy- Uridine-5'-triphosphate |
| 2'-azido-2'-deoxy- uridine-5'-triphosphate |
| 2'-fluoro-2'-deoxy- guanosine-5'-triphosphate |
| 2'-O-methyl-5-Methyl-uridine-5'-triphosphate |

### Working examples 1 to 14 and comparative example 15

The reactions mixtures for Examples 1 to 14 and Comparative Example 15 (including only unmodified rNTPs) were set up according to the following Table 2:

**Table 2: Reaction mixtures**

| **Examples: No. 1 bis 15** | | | | | | | | | | | | | | | |
|---|---|---|---|---|---|---|---|---|---|---|---|---|---|---|---|
| **Reagent** | **Reaction Mixture No.** | | | | | | | | | | | | | | |
| | **1** | **2** | **3** | **4** | **5** | **6** | **7** | **8** | **9** | **10** | **11** | **12** | **13** | **14** | **15 (comperative)** |
| Buffer (5-fold concentrate) | 10 µl | 10 µl | 10 µl | 10 µl | 10 µl | 10 µl | 10 µl | 10 µl | 10 µl | 10 µl | 10 µl | 10 µl | 10 µl | 10 µl | 10 µl |
| rATP (10 mM) | 2 µl | 2 µl | 2 µl | -- | 2 µl | -- | -- | 2 µl | 2 µl | 2 µl | 2 µl | 2 µl | 2 µl | 2 µl | 2 µl |
| rGTP (10 mM) | 2 µl | 2 µl | 2 µl | 2 µl | 2 µl | 2 µl | 2 µl | 2 µl | 2 µl | 2 µl | 2 µl | 2 µl | -- | 2 µl | 2 µl |
| rCTP (10 mM) | -- | 2 µl | 2 µl | 2 µl | 2 µl | 2 µl | 2 µl | -- | 2 µl | 2 µl | 2 µl | 2 µl | 2 µl | 2 µl | 2 µl |
| rUTP (10 mM) | 2 µl | -- | -- | 2 µl | -- | 2 µl | 2 µl | 2 µl | -- | -- | -- | -- | 2 µl | -- | 2 µl |
| 2'-O-Methyl-C | 2 µl | -- | -- | -- | -- | -- | -- | -- | -- | -- | -- | -- | -- | -- | -- |
| 5-Aminoallyl-U | -- | 2 µl | -- | -- | -- | -- | -- | -- | -- | -- | -- | -- | -- | -- | -- |
| 6-Aza-U | -- | -- | 2 µl | -- | -- | -- | -- | -- | -- | -- | -- | -- | -- | -- | -- |
| 8-Aza-A | -- | -- | -- | 2 µl | -- | -- | -- | -- | -- | -- | -- | -- | -- | -- | -- |
| 5-Bromo-U | -- | -- | -- | -- | 2 µl | -- | -- | -- | -- | -- | -- | -- | -- | -- | -- |
| 7-Deaza-A | -- | -- | -- | -- | -- | 2 µl -- | | -- | -- | -- | -- | -- | -- | -- | -- |
| N6-Methyl-A | -- | -- | -- | -- | -- | -- | 2 µl | -- | -- | -- | -- | -- | -- | -- | -- |
| 5-Methyl-C | -- | -- | -- | -- | -- | -- | -- | 2 µl | -- | -- | -- | -- | -- | -- | -- |
| Pseudo-U | -- | -- | -- | -- | -- | -- | -- | -- | 2 µl | -- | -- | -- | -- | -- | -- |
| 4-Thio-U | -- | -- | -- | -- | -- | -- | -- | -- | -- | 2 µl | -- | -- | -- | -- | -- |
| 2'-Amino-2'-deoxy-U | -- | | -- | -- | -- | -- | -- | -- | -- | -- | 2 µl | -- | -- | -- | -- |
| 2'-Azido-2'-deoxy-U | -- | -- | -- | -- | -- | -- | -- | -- | -- | -- | -- | 2 µl | -- | -- | -- |
| 2'-Fluoro-2'-deoxy-G | -- | -- | -- | -- | -- | -- | -- | -- | -- | -- | -- | -- | 2 µl | -- | -- |
| 2-O-Methyl-5-Methyl-U | -- | -- | -- | -- | -- | -- | -- | -- | -- | -- | -- | -- | -- | 2 µl | -- |
| Calicivirus RdRp | 5 µl | 5 µl | 5 µl | 5 µl | 5 µl | 5 µl | 5 µl | 5 µl | 5 µl | 5 µl | 5 µl | 5 µl | 5 µl | 5 µl | 5 µl |
| Aqua Dest | 26 µl | 26 µl | 26 µl | 26 µl | 26 µl | 26 µl | 26 µl | 26 µl | 26 µl | 26 µl | 26 µl | 26 µl | 26 µl | 26 µl | 26 µl |
| ssRNA-Template | 1 µl | 1 µl | 1 µl | 1 µl | 1 µl | 1 µl | 1 µl | 1 µl | 1 µl | 1 µl | 1 µl | 1 µl | 1 µl | 1 µl | 1 µl |

- buffer (5x): HEPES pH 7.6, 250 mM; MnCl2, 25 mM; DTT 5 mM
- ssRNA-template (1 µg/yl): heteropolymeric sequence, 19 to 25 nt length
- modified and/or unmodified ribonucleoside-triphosphate or deoxyribonulcleoside triphosphate were used as stock solutions of: 10 mM
- calicivirus RdRp: sapovirus RdRp (SEQ ID NO: 4) was used from stock solution of 70 µM

For RNA synthesis the reaction mixtures according to Examples 1 to 14 and Comparative Example 15 (no modified nucleotides present) were incubated at 30°C for 2 h.

The abbreviations used in Table 2 above stand for the following nucleotides as outlined in Table 3.

**Table 3: List of abbreviations for ribonucleoside triphosphates**

| **Abbreviation** | **Chemical Name** |
|---|---|
| rATP | adenosine-5'-triphosphate |
| rCTP | cytidine-5'-triphosphate |
| rGTP | guanosine-5'-triphosphate |
| rUTP | uridine-5'-triphosphate |
| 2'-O-Methyl-C | 2'-O-methyl-cytidine-5'-triphosphate |
| 5-Aminoallyl-U | 5-Aminoallyl-Uridine-5'-triphosphate |
| 6-Aza-U | 6-aza-uridine-5-'Triphosphate |
| 8-Aza-A | 8-aza-adenosine-5'-triphosphate |
| 5-Bromo-U | 5-bromo-uridine-5-'triphosphate |
| 7-Deaza-A | 7-deaza-adenosine-5'-triphosphate |
| 7-Deaza-G | 7-deaza- guanosine-5'-triphosphate |
| N6-Methyl-A | N⁶-methyl-adenosine-5'-triphosphate |
| 5-Methyl-C | 5-methyl- cytidine-5'-triphosphate |
| Pseudo-U | pseudo- uridine-5'-triphosphate |
| 4-Thio-U | 4-thio-uridine-5'-triphosphate |
| 2'-Amino-2'-deoxy-U | 2'-amino-2'-deoxy- uridine-5'-triphosphate |
| 2'-Azido-2'-deoxy-U | 2'-azido-2'-deoxy- uridine-5'-triphosphate |
| 2'-Fluoro-2'-deoxy-G | 2'-fluoro-2'-deoxy- quanosine-5'-triphosphate |
| 2-O-Methyl-5-Methyl-U | 2-O-methyl-5-methyl-uridine-5'-triphosphate |

### Synthesis of chemically modified and unmodified dsRNA using calicivirus RdRp

The reaction mixtures of Examples 1 to 14 and Comparative Example 15 were analysed by PAGE (polyacrylamide gel electrophoresis). Bands were detected by ethidium bromide staining. As shown in Fig. 1, the reaction mixtures of Examples 1 to 14 contain a species co-migrating with a 25 bp fragment of a dsRNA marker (lane M) which is also present in the reaction mixture of Comparative Example 15 in which only unmodified nucleotides were included in the reaction mixture.

These data suggest that RdRp of the *Caliciviridae* are capable of synthesizing dsRNA from various chemically modified building blocks.

### Purification and characterisation of chemically modified and unmodified dsRNA synthesised by calicivirus RdRp

The products of the reaction mixtures of Comparative Example 15 (unmodified dsRNA-Product), Example 1 (dsRNA-Product + 2-O-CH3-CMP) and Example 13 (dsRNA-Product + 2-F-dGMP) were purified by ion exchange (IEX) chromatography and fractionated. Elution profiles are shown in Fig. 2 and 3. The dashed lines show the fractions suspected to represent the dsRNA which were pooled and further analysed. The detection of the enzymatically synthesized (backbone-)modified (dsRNA-Product + 2-F-dGMP and dsRNA-Product + 2-O-CH3-CMP) or unmodified double-stranded RNA (dsRNA-Product) was performed with the pooled fractions by PAGE after staining with ethidium bromide and visualisation by UV-transillumination. For comparison, ssRNA template (ssRNA-Template in Fig. 2a and 2c) and a marker for dsRNA (lane M in Fig. 2c) were also loaded on the gel on separate lanes. The single-stranded RNA used as template for synthesis is visible as a lower band migrating at the level of the 17 bp band of the marker. The synthesized unmodified double-stranded RNA migrates at the level of the 25 bp band of the marker (dsRNA-Product). The synthesized 2'-O-methyl-cytidine-5'-phosphate backbone-modified double-stranded RNA migrates at the level of the 25 bp band of the marker (Fig. 3b, dsRNA-Product + 2-O-CH3-CMP) The synthesized 2'-fluoro-2'-deoxy-guanosine-5'-phosphate backbone-modified double-stranded RNA also migrates at the level of the 25 bp band of the marker (Fig. 3b, dsRNA-Product + 2-F-dGMP).

### Melting point analysis of chemically modified and unmodified dsRNA synthesised by calicivirus RdRp

The products of the reaction mixtures of Comparative Example 15 (unmodified dsRNA-Product), Example 1 (dsRNA-Product + 2-O-CH3-CMP) and Example 13 (dsRNA-Product + 2-F-dGMP) were incubated with a double-stranded RNA-intercalating agent (SYBR Green I) and the melting curves of the double-stranded RNA products were measured using the LightCycler device (Roche Diagnostics). The backbone-modified (dsRNA-Product + 2-O-CH3-CMP and dsRNA-Product + 2-F-dGMP, as indicated) and unmodified RNA (dsRNA-Product, as indicated) display various melting points as indicated in Fig. 3. These different melting curves result from incorporation of modified nucleotides in the RNA-backbone. Importantly, the single-stranded RNA template does not display a melting curve, being single stranded and hence not able to bind SYBR Green I. The shift in the melting points of the backbone-modified dsRNA products to the unmodified comparative dsRNA demonstrated that the RdRp has factually incorporated the modified nucleotides into the dsRNA.

### Mass spectrometric characterisation of an enzymatically synthesized double stranded RNA having 2'-O-CH3-CMP incorporation.

The products of the reaction mixture of enzymatic synthesis using 2'-O-CH₃-CTP (dsRNA product + 2-O-CH₃-CMP; Example 1) were analyzed by HPLC, with subsequent denaturation of the double stranded RNA leading to elution of the sense (template ssRNA) and antisense (complementary ssRNA) strands. The masses of the sense (template ssRNA, molecular weight of 7564.41) and the antisense (synthesized single stranded RNA complementary to the template ssRNA, molecular weight of 8080.73) were measured by ESI/MS. They were found to correspond exactly to the predicted mass of the template ssRNA and the incorporation of 2'-O-CH₃-CMP in the antisense strand (Figs. 5B and 5C).

Sequences of the sense (template) and antisense (product) strand:
ssRX21 for: GCUGAUGCCGUCAAGUUUACCCCC (SEQ ID NO: 7)
ssRX21 rev: 5-P₃-GGGGGUAAACUUGACGGCAUCAGC (SEQ ID NO: 8)

C residues in bold mark the 2'-O-CH₃-CMP residues.

Calculated molecular weights (Da): ssRX21 for: 7564,6; ssRX21 rev: 8080,9

MS-Analysis was carried out by LCMS using a standard HPLC system from Agilent Technologies, Inc. (Santa Clara, CA, USA), equipped with degasser, binary pump, temperature controllable autosampler, column oven and a DAD detector. The effluent from the DAD detector was coupled on-line with an accurate mass Q-TOF mass analyzer which was equipped with a dual spray electrospray source. An absolute amount of 10 mOD of total oligo was injected onto a reversed phase chromatography column and the oligonucleotides were separated using a methanol gradient.

## Claims

1. A method for enzymatically synthesizing chemically modified RNA comprising the steps of:
(a) providing ssRNA template; and
(b) interaction of the ssRNA template with a protein having RNA-dependent RNA polymerase (RdRp) activity under conditions sufficient for RNA synthesis in the presence of at least one ribonucleoside triphosphate having chemical modification at the ribose, phosphate and/or base moiety to provide chemically modified double-stranded RNA (dsRNA), wherein the chemical modification does not result in labelling of the dsRNA with a radioactive, fluorescent and/or chemical label used for detection of said dsRNA,
wherein the protein having RdRp activity has a "right hand conformation" and the amino acid sequence of the protein comprises the following sequence motifs:
a. XXDYS
b. GXPSG
c. YGDD
d. XXYGL
e. XXXXFLXRXX
with the following meanings:
D: aspartate
Y: tyrosine
S: serine
G: glycine
P: proline
L: leucine
F: phenylalanine
R: arginine
X: any amino acid.

2. The method of claim 1 wherein the protein is an RNA-dependent RNA polymerase of a virus of the *Caliciviridae* family.

3. The method of claim 2 wherein the protein is an RNA-dependent RNA polymerase of a noroviurs, sapovirus, vesivirus or lagovirus.

4. The method of claim 3 wherein the protein is selected from the group consisting of an RNA-dependent RNA polymerase of the norovirus strain (HuCV/NL/Dresden174/1997/GE (GenBank acc. No. AY741811), an RNA-dependent RNA polymerase of the sapovirus strain pJG-Sap01 (GenBank acc. No. AY694184), and an RNA-dependent RNA polymerase of the vesivirus strain FCV/Dresden/2006/GE (GenBank acc. No DQ424892).

5. The method of claim 4 wherein the protein has an amino acid sequence selected from the group consisting of SEQ ID NO: 1, SEQ ID NO: 2, SEQ ID NO: 3, SEQ ID NO: 4, SEQ ID NO 5: and SEQ ID NO: 6.

6. The method according to any one of the preceding claims further comprising
(c) separating the dsRNA into ssRNA.

7. The method method according to claim 6 further comprising performing the following steps (d) to (f):
(d) repeating steps (b) and (c) n times with n being an integer of at least 1;
(e) carrying out a final step (b); and
(f) stopping the reaction.

8. The method of claim 7 wherein n is an integer from 15 to 40.

9. The method of claim 6 or 7 wherein step (c) is carried out by heat denaturation, chemical denaturation or enzymatically.

10. The method according to any one of the claims 6 to 8 wherein the dsRNA is separated into ssRNA by a helicase.

11. The method according to any one of the preceding claims wherein the ssRNA template has a length of 15 to 30, preferably 21 to 28 nucleotides, more preferably 21 to 23 nucleotides.

12. The method according to any one of the preceding claims wherein the chemically modified dsRNA has an increased stability as compared to the non-modified analogue.

13. The method according to any one of the preceding claims wherein the at least one ribonucleoside triphosphate has a chemical modification at the ribose moiety.

14. The method of claim 13 wherein the 2'-OH group of the ribose moiety is replaced by a group selected from H, OR, R, halo, SH, SR, NH₂, NHR, NR₂ or CN, wherein R is C₁-C₆ alkyl, alkenyl or alkynyl and halo is F, Cl, Br or I.

15. The method of claim 14 wherein the at least one chemically modified ribonucleoside triphosphate is selected from the group consisting of 2'-O-methyl-cytidine-5'-triphosphate, 2'-amino-2'-deoxy-uridine, 2'-azido-2'-deoxyuridine-5'-triphosphate, 2'-fluoro-2'-deoxy-guanosine-5'-triphosphate and 2'-O-methyl-5-methyl-uridine-5'-triphosphate.

16. The method according to any one of the preceding claims wherein the at least one chemically modified ribonucleoside triphosphate has a chemical modification at the base moiety.

17. The method of claim 16 wherein the at least one chemically modified ribonucleoside triphosphate is selected from the group consisting of 5-aminoallyl-uridine-5'-triphosphate, 6-aza-uridine-5'-triphosphate, 8-aza-adenosine-5'-triphosphate, 5-bromo-uridine-5-'triphosphate, 7-deaza-adenosine-5'-triphosphate, 7-deaza-guanosine-5'-triphosphate, N⁶-methyl-adenosine-5'-triphosphate, 5-methyl- cytidine-5-'triphosphate, pseudo-uridine-5'-triphosphate, 4-thio-uridine-5'-triphosphate.

18. The method according to any one of the preceding claims wherein the at least one chemically modified ribonucleotide has a chemical modification at the phosphate moiety.

19. The method of claim 18 wherein the at least one chemically modified ribonucleoside triphosphate is a phosphothioate analogue.

20. A kit for carrying out the method according to any one of the preceding claims comprising:
- a protein having RdRp activity as defined in any one of claims 1 to 5;
- a buffer for providing conditions sufficient for RNA synthesis by the protein having RdRp activity as defined in any one of claims 1 to 5;
- rATP, rGTP, rCTP, rUTP;
- at least one ribonucleoside triphosphate having chemical modification at the ribose, phosphate and/or base moiety wherein the chemical modification does not provide a label for radioactive, fluorescent or chemical detection;
- a helicase.

21. The kit of claim 20 further comprising a stop solution.

## Patentansprüche

1. Verfahren zur enzymatischen Herstellung chemisch modifizierter RNA, umfassend die Schritte:
(a) Bereitstellen einer ssRNA-Matritze; und
(b) Wechselwirken der ssRNA-Matritze mit einem Protein mit RNA-abhängiger RNA-Polymerase (RdRp)-Aktivität unter Bedingungen, die zur RNA-Synthese geeignet sind, in Gegenwart von mindestens einem Ribonukleosid-Triphosphat mit einer chemischen Modifikation an der Ribose-, Phosphat- und/oder Basen-Einheit zur Erzeugung chemisch modifizierter doppelsträngiger RNA (dsRNA), wobei die chemische Modifikation nicht in einer Markierung der dsRNA mit einem radioaktiven, fluoreszierenden und/oder chemischen Marker zur Detektion der dsRNA resultiert,
wobei das Protein mit RdRp-Aktivität eine "Rechte Hand-Konformation" aufweist und die Aminosäure-Sequenz des Proteins die folgenden Sequenzmotive enthält:
a. XXDYS
b. GXPSG
c. YGDD
d. XXYGL
e. XXXXFLXRXX
mit den folgenden Bedeutungen:
D: Aspartat
Y: Tyrosin
S: Serin
G: Glycin
P: Prolin
L: Leucin
F: Phenylalanin
R: Arginin
X: eine beliebige Aminosäure.

2. Verfahren nach Anspruch 1, wobei das Protein eine RNA-abhängige RNA-Polymerase eines Virus der Familie der *Caliciviridae* ist.

3. Verfahren nach Anspruch 2, wobei das Protein eine RNA-abhängige RNA-Polymerase eines Norovirus, Sapovirus, Vesivirus oder Lagovirus ist.

4. Verfahren nach Anspruch 3, wobei das Protein aus der Gruppe, bestehend aus einer RNA-abhängigen RNA-Polymerase des Norovirus-Stamms HuCV/NL/Dresden174/1997/GE (GenBank Zugriffsnummer AY741811), einer RNA-abhängigen RNA-Polymerase des Sapovirus-Stamms pJG-Sap01 (GenBank Zugriffsnummer AY694184) und einer RNA-abhängigen RNA-Polymerase des Vesivirus-Stamms FCV/Dresden/2006/GE (GenBank Zugriffsnummer DQ424892), ausgewählt ist.

5. Verfahren nach Anspruch 4, wobei das Protein eine Aminosäure-Sequenz aufweist, die aus der Gruppe, bestehend aus SEQ ID NO: 1, SEQ ID NO: 2, SEQ ID NO: 3, SEQ ID NO: 4, SEQ ID NO: 5 und SEQ ID NO: 6, ausgewählt ist.

6. Verfahren nach einem der vorhergehenden Anprüche, das weiterhin
(c) das Auftrennen der dsRNA in ssRNA
umfasst.

7. Verfahren nach Anspruch 6, das weiterhin die Durchführung der folgenden Schritte (d) bis (f) umfasst:
(d) n-maliges Wiederholen der Schritte (b) und (c), wobei n eine ganze Zahl von mindestens 1 ist;
(e) Ausführen eines letzten Schrittes (b); und
(f) Stoppen der Reaktion.

8. Verfahren nach Anspruch 7, wobei n eine ganze Zahl zwischen 15 und 40 ist.

9. Verfahren nach Anspruch 6 oder 7, wobei Schritt (c) durch Hitzedenaturierung, chemische Denaturierung oder enzymatisch ausgeführt wird.

10. Verfahren nach einem der Ansprüche 6 bis 8, wobei die dsRNA durch eine Helikase in ssRNA aufgetrennt wird.

11. Verfahren nach einem der vorhergehenden Ansprüche, wobei die ssRNA-Matritze eine Länge von 15 bis 30, bevorzugt 21 bis 28 Nukleotiden, mehr bevorzugt 21 bis 23 Nukleotiden aufweist.

12. Verfahren nach einem der vorhergehenden Ansprüche, wobei die chemisch modifizierte dsRNA eine erhöhte Stabilität im Vergleich zu dem nichtmodifizierten Analogon aufweist.

13. Verfahren nach einem der vorhergehenden Ansprüche, wobei das mindestens eine Ribonukleosid-Triphosphat eine chemische Modifikation an der Ribose-Einheit aufweist.

14. Verfahren nach Anspruch 13, wobei der 2'-OH-Rest der Ribose-Einheit durch einen Rest ausgewählt aus H, OR, R, Halo, SH, SR, NH₂, NHR, NR₂ oder CN ausgewählt ist, wobei R C₁- bis C₆-Alkyl, -Alkenyl oder -Alkynyl ist, und Halo F, Cl, Br oder I ist.

15. Verfahren nach Anspruch 14, wobei das mindestens eine chemisch modifizierte Ribonukleosid-Triphosphat aus der Gruppe, bestehend aus 2'-O-Methyl-cytidin-5'-triphosphat, 2'-Amino-2'-deoxy-uridin-5'-triphosphat, 2'-Azido-2-deoxyuridin-5'-triphosphat, 2'-Fluor-2'-deoxyguanosin-5'-triphosphat und 2'-O-Methyl-5-methyl-uridin-5'-triphosphat, ausgewählt ist.

16. Verfahren nach einem der vorhergehenden Ansprüche, wobei das mindestens eine chemisch modifizierte Ribonukleosid-Triphosphat eine chemische Modifikation an der Basen-Einheit aufweist.

17. Verfahren nach Anspruch 16, wobei das mindestens eine chemisch modifizierte Ribonukleosid-Triphosphat aus der Gruppe, bestehend aus 5-Aminoallyl-uridin-5'-triphosphat, 6-Aza-uridin-5'-triphosphat, 8-Aza-adenosin-5'-triphosphat, 5-Brom-uridin-5'-triphosphat, 7-Deaza-adenosin-5'-triphosphat, 7-Deaza-guanosin-5'-triphosphat, N⁶-Methyl-adenosin-5'-triphosphat, 5-Methyl-cytidin-5'-triphosphat, Pseudo-uridin-5'-triphosphat, 4-Thio-uridin-5'-triphosphat, ausgewählt ist.

18. Verfahren nach einem der vorhergehenden Ansprüche, wobei das mindestens eine chemisch modifizierte Ribonukleotid eine chemische Modifikation an der Phosphat-Einheit aufweist.

19. Verfahren nach Anspruch 18, wobei das mindestens eine chemisch modifizierte Ribonukleosid-Triphosphat ein Phosphothioat-Analogon ist.

20. Kit zur Durchführung des Verfahrens nach einem der vorhergehenden Ansprüche, das
- ein Protein mit RdRp-Aktivität gemäß der Definition in einem der Ansprüche 1 bis 5,
- einen Puffer zur Bereitstellung von Bedingungen, die zur RNA-Synthese mit einem Protein mit RdRp-Aktivität gemäß der Definition in einem der Ansprüche 1 bis 5, geeignet sind,
- rATP, rGTP, rCTP, rUTP,
- mindestens ein Ribonukleosid-Triphosphat mit einer chemischen Modifikation an der Ribose-, Phosphat- und/oder Basen-Einheit, wobei die chemische Modifikation keinen Marker zur radioaktiven, Fluoreszenz- oder chemischen Detektion bereitstellt,
- eine Helikase
umfasst.

21. Kit nach Anspruch 20, das weiterhin eine Stop-Lösung umfasst.

## Revendications

1. Procédé de synthèse enzymatique d'ARN chimiquement modifié comprenant les étapes suivantes :
(a) la fourniture de matrice d'ARNsb; et
(b) l'interaction de la matrice d'ARNsb avec une protéine possédant une activité ARN polymérase ARN-dépendante (RdRp) dans des conditions suffisantes pour la synthèse d'ARN en présence d'au moins un ribonucléoside triphosphate présentant une modification chimique niveau du radical ribose, phosphate et/ou base pour fournir de l'ARN double brin (ARNdb) chimiquement modifié, où la modification chimique n'entraîne pas de marquage de l'ARNdb avec un marqueur radioactif, fluorescent et/ou chimique utilisé pour la détection dudit ARNdb,
dans lequel la protéine possédant une activité RdRp présente une « conformation main droite » et la séquence d'acides aminés de la protéine comprend les motifs de séquence suivants ;
a. XXDYS
b. GXPSG
c. YGDD
d. XXYGL
e. XXXXFLXRXX
avec les significations suivantes :
D : aspartate
Y : tyrosine
S : sérine
G : glycine
P : proline
L : leucine
F : phénylalanine
R : arginine
X : un acide aminé quelconque.

2. Procédé selon la revendication 1, dans lequel la protéine est une ARN polymérase ARN-dépendante d'un virus de la famille des *Caliciviridae*.

3. Procédé selon la revendication 2, dans lequel la protéine est une ARN polymérase ARN-dépendante d'un norovirus, d'un sapovirus, d'un vésivirus ou d'un lagovirus.

4. Procédé selon la revendication 3, dans lequel la protéine est choisie dans le groupe constitué par une ARN polymérase ARN-dépendante de la souche de norovirus HuCV/NL/Dresden174/1997/GE (No. d'accession GenBank AY741811), une ARN polymérase ARN-dépendante de la souche de sapovirus pJG-Sap01 (No. d'accession GenBank AY694184) et une ARN polymérase ARN-dépendante de la souche de vésivirus FCV/Dresden/2006/GE (No. accession GenBank DQ424892).

5. Procédé selon la revendication 4, dans lequel la protéine a une séquence d'acides aminés choisie dans le groupe constitué de SEQ ID NO : 1, SEQ ID NO : 2, SEQ ID NO : 3, SEQ ID NO : 4, SEQ ID NO : 5 et SEQ ID NO : 6.

6. Procédé selon l'une quelconque des revendications précédentes, comprenant en outre
(c) la séparation de l'ARNdb en ARNsb.

7. Procédé selon la revendication 6, comprenant en outre la réalisation des étapes (d) à (f) suivantes :
(d) la répétition des étapes (b) et (c) n fois avec n étant un nombre entier d'au moins 1 ;
(e) la réalisation d'une étape (b) finale ; et
(f) l'arrêt de la réaction.

8. Procédé selon la revendication 7, dans lequel n est un nombre entier de 15 à 40.

9. Procédé selon la revendication 6 ou 7, dans lequel l'étape (c) est réalisée par une dénaturation par la chaleur, une dénaturation chimique ou par voie enzymatique.

10. Procédé selon l'une quelconque des revendications 6 à 8, dans lequel l'ARNdb est séparé en ARNsb par une hélicase.

11. Procédé selon l'une quelconque des revendications précédentes, dans lequel la matrice d'ARNsb a une longueur de 15 à 30, de préférence 21 à 28 nucléotides, de manière davantage préférée 21 à 23 nucléotides.

12. Procédé selon l'une quelconque des revendications précédentes, dans lequel l'ARNdb chimiquement modifié présente une stabilité accrue comparativement à l'analogue non modifié.

13. Procédé selon l'une quelconque des revendications précédentes, dans lequel le au moins un ribonucléoside triphosphate présente une modification chimique au niveau du radical ribose.

14. Procédé selon la revendication 13, où le groupe 2'-OH du radical ribose est remplacé par un groupe choisi parmi H, OR, R, halogéno, SH, SR, NH₂, NHR, NR₂ ou CN, où R représente un groupe alkyle alcényle ou alcynyle en C₁ à C₆, et halogéno est F, Cl, Br ou I.

15. Procédé selon la revendication 14, dans lequel le au moins un ribonucléoside triphosphate chimiquement modifié est choisi dans le groupe constitué de 2'-O-méthyl-cytidine-5'-triphosphate, 2'-amino-2'-désoxy-uridine, 2'-azido-2'-désoxy-uridine-5'-triphosphate, 2'-fluoro-2'-désoxy-guanosine-5'-triphosphate et 2'-O-méthyl-5-méthyl-uridine-5'-triphosphate.

16. Procédé selon l'une quelconque des revendications précédentes, dans lequel le au moins un ribonucléoside triphosphate chimiquement modifié présente une modification chimique au niveau du radical base.

17. Procédé selon la revendication 16, dans lequel le au moins un ribonucléoside triphosphate chimiquement modifié est choisi dans le groupe constitué de 5-aminoallyl-uridine-5'-triphosphate, 6-aza-uridine-5'-triphosphate, 8-aza-adénosine-5'-triphosphate, 5-bromo-uridine-5'-triphosphate, 7-déaza-adénosine-5'-triphosphate, 7-déaza-guanosine-5'-triphosphate, N⁶-méthyl-adénosine-5'-triphosphate, 5-méthyl-cytidine-5'-triphosphate, pseudo-uridine-5'-triphosphate, 4-thio-uridine-5'-triphosphate.

18. Procédé selon l'une quelconque des revendications précédentes, dans lequel le au moins un ribonucléotide chimiquement modifié présente une modification chimique au niveau du radical phosphate.

19. Procédé selon la revendication 18, dans lequel le au moins un ribonucléoside triphosphate chimiquement modifié est un analogue phosphothioate.

20. Kit permettant de réaliser le procédé selon l'une quelconque des revendications précédentes, comprenant :
- une protéine possédant une activité RdRp telle que définie dans l'une quelconque des revendications 1 à 5 ;
- un tampon pour fournir des conditions suffisantes pour la synthèse d'ARN par la protéine possédant une activité RdRp telle que définie dans l'une quelconque des revendications 1 à 5 ;
- rATP, rGTP, rCTP, rUTP ;
- au moins un ribonucléoside triphosphate présentant une modification chimique au niveau du radical ribose, phosphate et/ou base, où la modification chimique ne fournit pas de marqueur pour une détection radioactive, fluorescente ou chimique ;
- une hélicase.

21. Kit selon la revendication 20, comprenant en outre une solution d'arrêt.
